# EUROPEAN PATENT SPECIFICATION

(11) **EP 4 364 698 B1**
(45) Date of publication and mention of the grant of the patent: **11.02.2026**
(21) Application number: 23206857.7
(22) Date of filing: 30.10.2023
(51) Int. Cl.: A61F 2/30, A61F 2/34

(54) **PELVIC IMPLANTATION DEVICE**
BECKENIMPLANTATIONSVORRICHTUNG
DISPOSITIF D'IMPLANTATION PELVIENNE

(30) Priority: 02.11.2022 US 202263381950 P
(43) Date of publication of application: 08.05.2024
(73) Proprietor: Waldemar Link GmbH & Co. KG, 22339 Hamburg (DE)
(72) Inventor: LINK, Helmut D., Hamburg (DE); FISCHER, Hans-Joachim, Norderstedt (DE)
(74) Representative: Alatis

(56) References cited:
- DE-A1- 19 901 710
- FR-A1- 2 784 289
- US-A1- 2021 077 262
- US-A1- 2022 226 121

## Description

### BACKGROUND

Hip joints may suffer degeneration through disease or through wear. Hip joint endoprostheses have proven useful for therapy. These comprise a femoral component, which is implanted at the upper end of the thigh bone, and a hip component, which interacts with the femoral component and is implanted in the pelvic bone (acetabulum). In many cases, it can happen that the pelvic bone is damaged in the region of the hip joint. This has the effect of making it difficult to fasten the hip joint component to the pelvic bone. This is especially true of implants that function as a partial replacement of the pelvis. These have additional fastening elements in order not only to achieve secure anchoring of the joint socket of the hip joint implant on the pelvic bone, but also to stabilize the pelvic bone itself.

Some hip joint implants of this kind are known, they have a plurality of fastening elements on a support body that comprises the actual joint socket. These fastening elements comprise elongate brackets which extend in a cranial direction and which are provided with a plurality of drilled holes to permit fastening by means of a bone screw. Moreover, opposite these fastening brackets, a caudally directed and shorter bracket is provided, which can optionally also be configured like a hook. They serve to securely fasten and stabilize the implant, and in particular the joint socket thereof, on a damaged pelvic bone.

A tried and tested material for implants is TiAl6V4 which, although sufficiently robust, nevertheless has the disadvantage that it cannot be reshaped during surgery. Pure titanium, by contrast, is much easier to reshape, but, due to a lack of dimensional stability, it is not routinely used for the pelvic socket component. This conflict of objectives has not hitherto been able to be satisfactorily resolved.

The object of the disclosure is to create an improved hip joint implant that avoids these disadvantages and others. The disclosure moreover extends to a corresponding production method.

US 2021/0077262 A1 discloses prostheses, acetabular apparatuses, and methods of use of such prostheses and apparatuses. In some embodiments, an acetabular apparatus includes an acetabular cup, a pre-assembled liner and flange construct, and a dual mobility bearing (e.g., a dual articulating femoral head component and insert). In one embodiment, the pre-assembled liner and flange construct may be coupled together by a band coupled to the flanges, the band being arranged and configured to be pressed onto an outer surface of a liner. The pre-assembled liner and flange construct is arranged and configured to accept the dual mobility bearing so that it is freely rotatable relative thereto during use.

US 2022/0226121 discloses a hip joint implant for fastening to a pelvic bone, with a support body which has a socket and whose convex outer face is designed to bear on the pelvic bone and which, on its concave inner face, has a receiving seat for a pelvis-side bearing that is designed to receive a joint head of a femoral component of a hip prosthesis, and with outwardly directed flat fastening brackets which are arranged at the edge region of the socket and are each provided with at least one receiving seat for a fastening means. The fastening brackets are made of a reshapeable biocompatible material and are connected to the socket via a non-releasable cohesive bond. The socket is made of another, stiffer biocompatible material. A high degree of robustness of the socket is thus combined with what is, by virtue of the reshapeability, an improved adaptation to the anatomical conditions of the respective pelvic bone. This improves reliability of fastening, stability and long-term behavior.

### SUMMARY

A pelvic implantation device according to the invention is defined in claim 1.

In a hip joint implant for fastening to a pelvic bone, with a support body which has a socket and whose convex outer face is designed to bear on the pelvic bone and which, on its concave inner face, has a receiving seat for a pelvis-side bearing component that is designed to receive a joint head of a femoral component of a hip prosthesis, and with outwardly directed flat fastening brackets which are arranged at the edge region of the socket and are each provided with at least one receiving seat for a fastening means.

In some embodiments of the present disclosure, for a disclosed hip joint implant, provision is made according to the disclosure that the fastening brackets are made of a reshapeable (cold-formable) substantially biocompatible material and are connected to the socket via a cohesive bond, wherein the socket is made of another, stiffer substantially biocompatible material.

As used herein, the term "reshapeable" is understood to mean a material that is substantially plastically deformable at room temperature, such that fastening brackets formed from this material can be manually adapted, with or without hand tools, to an underlying structure. This disclosure also refers to these abilities of a material as "cold-formable".

In contrast to "reshapeable" or "cold-formable", "non-cold-formable" material identifies material that cannot be plastically deformed manually, or by using hand tools, at room temperature.

As used herein, the term "biocompatible" or "biocompatible material" refers to any material which upon implantation into a mammal's body that does not elicit a substantial detrimental response *in vivo,* and/or a material exhibiting essentially no cytotoxicity or immunogenicity while in contact with body fluids, tissues and/or bones. For example, the biocompatible material can be selected from the group consisting of titanium (of any grade), Nitinol^{®}, steel, surgical steel, calcium, copper, zinc, iron, cobalt, magnesium, manganese, vanadium, molybdenum, silicate, strontium, tungsten, chromium, nickel, aluminum, and ceramics, composites, alloys (such as TiA16V4), compounds, and mixtures thereof.

The bearing component can be designed as an insert which is made of a material that promotes sliding and which functions as a receiving seat and sliding partner for a joint head. Such an insert is usually fastened by being cemented or clamped in the support body, but other types of fastening can also be provided. The bearing component can also be designed, however, as an interposition component. This is usually understood to mean an intermediate piece of this type that changes an opening angle of the socket, in particular for adaptation to particular anatomical conditions. The element forming the sliding partner for the joint head is then received in this intermediate piece. Moreover, the bearing component can also be introduced into and fixed in the support body as a dual mobility insert.

The disclosure includes the concept of a division of material, in the sense that different materials are used for the fastening brackets and the socket-like support body, and these materials can be cohesively connected, and therefore can be permanently connected, to one another. The fastening brackets are produced from a reshapeable, biocompatible material, for example titanium (for example of grade 2, grade 3 or grade 4, however, any suitable grade can solely be used, or be included in combination with other grades and/or other materials). This material can possibly, at least partially, be reshaped intraoperatively by the surgeon, so that the support body that is ultimately implanted can achieve a relatively good adaptation to the respective individual peculiarities of the pelvic bone of the respective patient.

By contrast, for the support body with the socket, which by means of a bearing component forms the bearing for the joint head of the femoral component, another biocompatible material is used, for example a titanium alloy such as TiA16V4. This material is stiffer as compared to the fastening bracket material, cost-effective, mechanically robust and only marginally reshapeable. However, the latter is not wholly necessary for the socket; on the contrary, the lack of reshapeability increases the robustness and dimensional accuracy of the socket, even under relatively high loads. By virtue of the dimensional accuracy, the construction according to the disclosure is particularly suitable for sockets of modular design, with alternative sockets and socket inserts being used if necessary.

The disclosure has recognized that the hitherto unresolved conflict between a robust and cost-effective implant, on the one hand, and good reshapeability for better adaptation and fastening to the individual shape of the pelvic bone, on the other hand, can be resolved.

The cohesive bonds of the present disclosure can be a welded connection, in particular as an electron beam welded connection. This affords the advantage of a high degree of reproducibility and a good welding result with relatively minimal heat input. As a result of the relatively low heat input, there is also only a small amount of distortion. Furthermore, it permits the welding of various materials to each other, for example titanium with titanium alloys. This connection also permits a high degree of automation, which likewise benefits the reproducibility and therefore the quality of the connection as a whole.

The cohesive bond is advantageously designed with a welded-through weld seam. A square butt weld, which permits reliable fastening and safe through-welding with little heat input, can be used. However, the cohesive bond can be formed through any other welding process and/or any adhesive material addition.

The fastening brackets can be configured individually and separately from the fastening ring. However, in the context of the present disclosure, the fastening brackets are designed and described as being in one piece with a fastening ring, which engages around the socket-like support body and is connected to the socket by means of the cohesive bond. This provides for good pre-assembly, namely of the fastening brackets to the fastening ring. After this has been prefabricated (and, if necessary, reworked), it can be pre-assembled with the socket-like support body.

The fastening brackets are prefabricated with openings for fastening means. In some embodiments, these will be drilled or formed holes that are configured to receive fastening screws (such as bone screws) for fastening the bracket to the pelvic bone. Furthermore, the fastening ring can be prefabricated to the extent that it has an opening which is dimensioned in such a way that it functions to receive the socket-like support body. The opening is preferably prefabricated to a dimension that corresponds to an external dimension of the support body, in particular to an external diameter of the socket of the support body. Advantageously, the opening in the fastening ring can be configured with a corresponding dimension in such a way that it forms an interference fit with the socket-like support body. This permits an initial fixing, which can be a step of pre-assembly.

The socket itself can be made of a non-cold-formable material, for example any material that is less deformable than the socket-like support body, such as titanium (of any grade), Nitinol^{®}, steel, surgical steel, calcium, copper, zinc, iron, cobalt, magnesium, manganese, vanadium, molybdenum, silicate, strontium, tungsten, chromium, nickel, aluminum, and ceramics, composites, alloys (such as TiA16V4), compounds, and mixtures thereof. As one example, an alloy of TiA16V4 that achieves a tensile strength of 800 MPa or greater can be used, with titanium itself achieving a tensile strength of ca. 300 MPa.

The socket-like support body can be provided with a support shoulder and/or a centering ring in the region where the fastening ring is received. The support shoulder can be designed entirely or partially circumferentially. The support shoulder can thus form a stop for the fastening ring, such that the position of the latter is defined when the fastening ring is pushed onto the socket. The centering ring can aid in positioning the fastening ring in a substantially central position. The centering ring can also serve as an interface for a modular system. This makes it possible to use other types of sockets too, for example sockets that belong to a modular system consisting of several components. These can also include sockets that are designed differently but have a uniform external diameter. This is especially true when several uniform external diameters are provided, which are graduated in different external diameter sizes.

The transition region between the socket and fastening brackets or fastening ring can be provided with a milled surface. This can be applied, for example, as part of secondary processing after the cohesive bonding. On the one hand, this makes the fastening ring smaller and, on the other hand, the surface quality is improved.

The disclosure further relates to a method for producing, and a method of implanting, a corresponding hip joint implant.

### BRIEF DESCRIPTION OF THE DRAWINGS

The accompanying drawings, which are incorporated in and constitute a part of this specification, illustrate embodiments of the disclosure, and, together with the summary given above, and the detailed description of the embodiments below, serve as a further explanation and disclosure to explain and/or illustrate embodiments of the disclosure.
FIG. 1 shows an example of a hip joint implant in the state when fitted on a pelvic bone;
FIG. 2A is a view of a fastening ring with fastening brackets;
FIG. 2B is a view of a fastening ring with fastening brackets of an embodiment of the invention;
FIG. 3A is a perspective view of a socket-like support body;
FIGs. 3B and 3C are perspective views of a socket-like support body;
FIG. 4A is a perspective view of an inserted configuration of the socket-like support body;
FIG. 4B is a perspective view of an inserted configuration of the socket-like support body;
FIG. 5A is a perspective view of reshaped fastening brackets and fastening ring, with the socket-like support body in an inserted configuration;
FIG. 5B is a perspective view of a reshaped fastening brackets and fastening ring, with the socket-like support body in an inserted configuration; and
FIG. 5C is a perspective view of reshaped fastening brackets and fastening ring, with the socket-like support body in an inserted configuration, with a reshaped fastening brackets and fastening ring overlaid on the reshaped fastening brackets and fastening ring.

### DETAILED DESCRIPTION

It is noted that the drawings of the present application are provided for illustrative purposes only and, as such, the drawings are not drawn to scale. It is also noted that like and corresponding elements are referred to by like reference numerals.

In the following description, numerous specific details are set forth, such as particular structures, components, materials, dimensions, processing steps and techniques, in order to provide an understanding of the various embodiments of the present application. However, it will be appreciated by one of ordinary skill in the art that various embodiments of the present application may be practiced without these specific details. In other instances, well-known structures or processing steps have not been described in detail in order to avoid obscuring the present application.

As used herein, the term "substantially" or "substantial", is equally applicable when used in a negative connotation to refer to the complete or near complete lack of an action, characteristic, property, state, structure, item, or result. For example, a surface that is "substantially" flat would either be completely at, or so nearly flat that the effect would be the same as if it were completely flat.

As used herein, terms defined in the singular are intended to include those terms defined in the plural and vice versa.

As used in this specification and its appended claims, terms such as "a", "an" and "the" are not intended to refer to only a singular entity, but include the general class of which a specific example may be used for illustration, unless the context dictates otherwise. The terminology herein is used to describe specific embodiments of the disclosure, but their usage does not delimit the disclosure, except as outlined in the claims.

Unless otherwise indicated, all numbers expressing quantities of ingredients, properties such as molecular weights, reaction conditions, and so forth as used in the specification and claims are to be understood as being modified in all instances by the term "about." Accordingly, unless indicated to the contrary, the numerical parameters in the specification and claims are approximations that may vary depending upon the desired properties sought to be obtained by the present disclosure. Each numerical parameter should at least be construed in light of the number of reported significant digits and by applying ordinary rounding techniques. Notwithstanding that the numerical ranges and parameters describing the broad scope of the disclosure are approximations, the numerical values in the specific examples are reported as precisely as possible. Any numerical value, however, inherently contains standard deviations that necessarily result from the errors found in the numerical value's testing measurements.

Thus, reference herein to any numerical range expressly includes each numerical value (including fractional numbers and whole numbers) encompassed by that range. To illustrate, reference herein to a range of "at least 50" or "at least about 50" includes whole numbers of 50, 51, 52, 53, 54, 55, 56, 57, 58, 59, 60, etc., and fractional numbers 50.1, 50.2 50.3, 50.4, 50.5, 50.6, 50.7, 50.8, 50.9, etc. In a further illustration, reference herein to a range of "less than 50" or "less than about 50" includes whole numbers 49, 48, 47, 46, 45, 44, 43, 42, 41, 40, etc., and fractional numbers 49.9, 49.8, 49.7, 49.6, 49.5, 49.4, 49.3, 49.2, 49.1, 49.0, etc. In yet another illustration, reference herein to a range of from "5 to 10" includes whole numbers of 5, 6, 7, 8, 9, and 10, and fractional numbers 5.1, 5.2, 5.3, 5,4, 5,5, 5.6, 5.7, 5.8, 5.9, etc.

In the discussion and claims herein, the tern "about" indicates that the value listed may be somewhat altered, as long as the alteration does not result in nonconformance of the process or structure to the illustrated embodiment. For example, for some elements the term "about" can refer to a variation of +0.1%, for other elements, the term "about" can refer to a variation of ±1% or ±10%, or any point therein.

Reference now will be made in detail to embodiments of the disclosure. It will be apparent to those skilled in the art that various modifications and variations can be made in the present disclosure. For instance, features illustrated or described as part of one embodiment can be used on another embodiment to yield a still further embodiment.

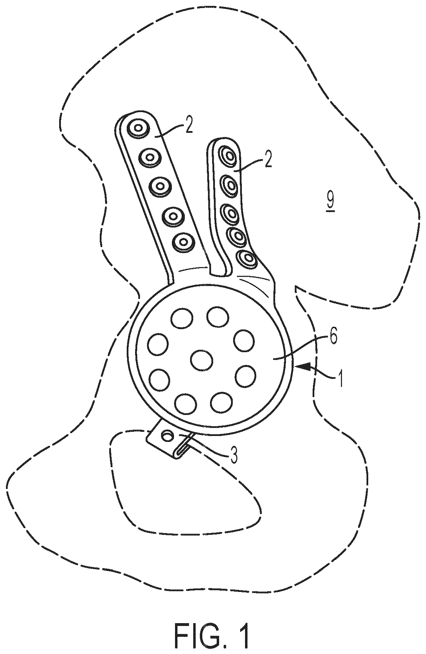

An illustrative example of a hip joint implant is shown in FIG. 1 in a state when fitted on a pelvic bone 9 (acetabulum).

In this example, the hip joint implant is designated as a whole by reference number 1 and comprises a socket-like support body 6 on which two flat and elongate fastening brackets 2 are arranged pointing upward (cranially). A shorter fastening bracket 3, extending downward (caudally), is provided on the opposite side of the socket-like support body 6 (in the following also called socket for short).

In another example described in this application, the location and/or use of a hip joint implant 100 can be the same as the hip joint implant 1's location in FIG. 1.

In the illustrative example shown, the implant 1 is composed of two components, a socket 6 and a fastening component, which comprises the fastening brackets 2, 3 and a fastening ring 4, on which the fastening brackets 2, 3 are arranged. In embodiments of the invention, the implant 1 can include a socket 106, which is further discussed below.

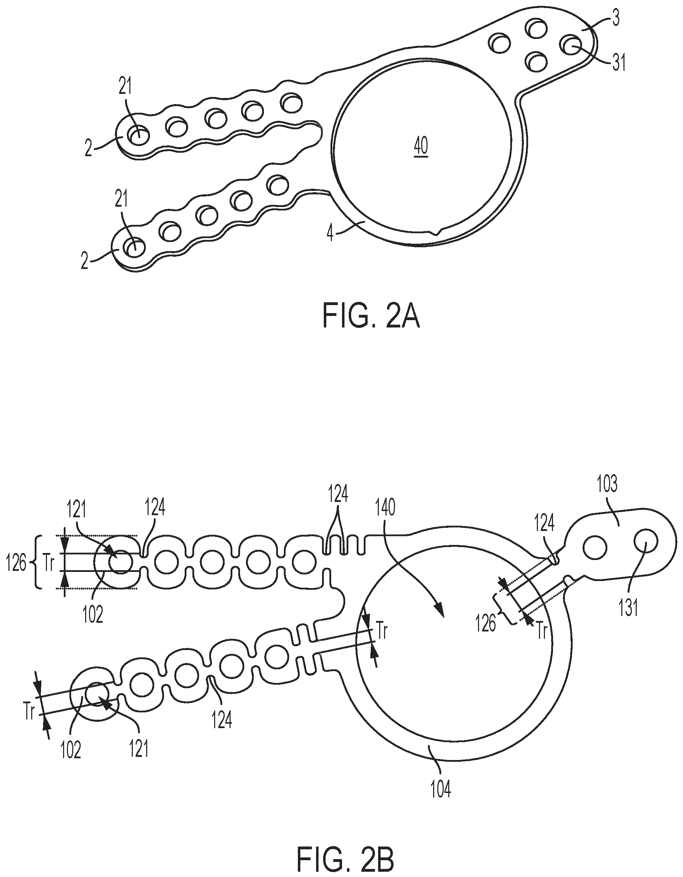

In FIG. 1, the fastening brackets 2 are each substantially elongate, substantially flat elements that are provided with a plurality of openings 21 (shown in FIG. 2A) configured to receive fastening means. The fastening bracket 3 is a substantially flat element, but, in the illustrative example shown, it is shorter and wider than the fastening brackets 2. This is only an example; the disclosure is not limited thereto. The fastening bracket 3 is likewise provided with a plurality of openings 31 (shown in FIG. 2A) configured to receive fastening means. The openings 21, 31 are designed as through-openings configured to receive fastening means (not shown). Screws, such as bone screws are one example of fastening means, however, any surgical fastening device can be used.

Reference is now made to FIG. 2A. The fastening brackets 2, 3 are arranged protruding radially outward on a ring 4, which is designated as a fastening ring 4. In this example shown, the fastening brackets 2, 3 and the fastening ring 4 are made in one piece, however, in other examples the fastening brackets 2, 3 and the fastening ring 4 are two or more pieces joined together in any suitable way. The fastening brackets 2, 3 and the fastening ring 4 are made of a biocompatible material, which is relatively easily deformed manually. It is therefore possible for the operator to at least partially reshape the fastening brackets 2, 3 even during the implantation, i.e., during the operation, if necessary, and thus better adapt them to the particular anatomical features of the pelvic bone 9 of the respective patient.

In FIG. 2A, the fastening ring 4 delimits an inner region 40 which is designed to receive the socket 6 (or a socket 106 described below). In the illustrative example shown in FIG. 2A, the fastening ring 4 is designed fully circumferentially, although this is not necessary, the ring does not need to be completely closed and in other examples the fastening ring 4 can be partially open.

In FIG. 2B, which illustrates an embodiment of the invention, fastening brackets 102 are each substantially elongate, substantially flat elements that are provided with a plurality of openings 121configured to receive fastening means. Fastening bracket 103 is a substantially flat element, but, in the illustrative embodiment shown, it is shorter and wider than the fastening brackets 102. This embodiment is only an example; the disclosure is not limited thereto. The fastening bracket 103 is likewise provided with a plurality of openings 131 configured to receive fastening means. The openings 121, 131 are designed as through-openings configured to receive fastening means (not shown). Screws, such as bone screws are one example of fastening means, however, any surgical fastening device can be used.

Fastening brackets 102 and 103 are arranged protruding radially outward on a ring 104, which is designated as a fastening ring 104. In this embodiment shown, fastening brackets 102, and 103 and the fastening ring 104 are one piece, however, in other embodiments the fastening brackets 102, 103 and the fastening ring 4 are two or more pieces joined together in any suitable way.

In FIG. 2B, the fastening ring 104 delimits an inner region 140 which is designed to receive the socket 6 (or a socket 106 described below). In the illustrative embodiment shown in FIG. 2B, the fastening ring 104 is designed fully circumferentially, although this is not necessary, the ring does not need to be completely closed and in other embodiments the fastening ring 104 can be partially open.

The fastening brackets 102, 103 and the fastening ring 104 can be made of a biocompatible material, which is relatively easily deformed manually. Alternatively, or in addition to the biocompatible material, the fastening brackets 102, 103 and the fastening ring 104 can be made of any suitable material that can substantially maintain a shape upon deformation, such as metal(s) (such as titanium aluminum, magnesium, etc. and combinations thereof), plastic(s), epoxy(s), rubber(s), cellulose based material(s), organic material(s) such as leather(s), fabric(s)(woven and/or non-woven), carbon based material(s), and combinations thereof.

Thus, it is therefore possible for the operator to relatively easily, and comparatively easier than reshaping of the fastening brackets 2, 3 and the fastening ring 4, at least partially reshape the fastening brackets 102, 103 and the fastening ring 104 prior to or even during the implantation, i.e. during the operation, if necessary. This relative ease of reshaping the fastening brackets 102, 103 and the fastening ring 104 is due to one or more of (1) the higher workability and ease of reshaping of the material of the fastening brackets 102, 103 and the fastening ring 104, and/or (2) inclusion of narrow portions 124 in each of the fastening brackets 102, 103 and the fastening ring 104. As can be seen in FIG. 2B, the narrow portions 124 are relatively narrower than a width dimension 126 of each of the fastening brackets 102, 103 and the fastening ring 104. Due to the narrow portions 124 being relatively narrower than the width dimension 126s, and therefore composed of less material to be reshaped, manual reshaping of the fastening brackets 102, 103 and the fastening ring 104 can occur. The narrow portions 124 can be included in between one, two or more, or all of the adjacent openings 121 of the fastening brackets 102, 103. Additional narrow portions 124 can be included between the opening 121 nearest the inner region 140 and the fastening ring 104. As can be seen in FIG. 2B, the narrow portion nearest the inner region 140 can be any area of less material as compared to the width dimension 126.

This reshaping of the fastening brackets 102, 103 and the fastening ring 104 can both (1) better adapt the fastening brackets 102, 103 and the fastening ring 104 to the particular anatomical features of the pelvic bone 9 of the respective patient, and (2) provide a model shape so that once the fastening brackets 102, 103 and the fastening ring 104 are removed from contacting the patient's pelvic bone 9, the fastening brackets 102, 103 and the fastening ring 104 can provide a model for reshaping of a to be implanted fastening brackets 2, 3 and fastening ring 4.

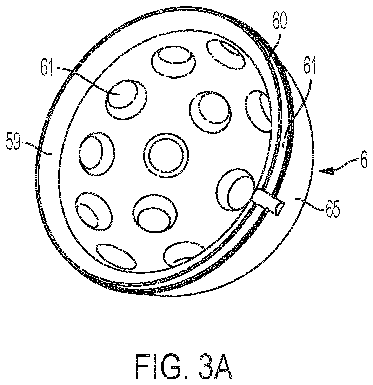

The socket 6 (or socket-like support body 6) is shown in FIG. 3A has a substantially hemispherical shape. The socket 6 includes a curved, dome shape and is provided with a plurality of openings 61. On its convex outer face 65, the socket-like support body 6 is configured to bear against the bone surfaces of the pelvic bone 9 and for this purpose can be provided with a surface or coating that promotes the incorporation of bone substance. On its inner face, the socket-like support body 6 is correspondingly concave and, together with a bearing component, forms the receiving seat for a joint head (not shown) of a femoral component of a hip joint prosthesis.

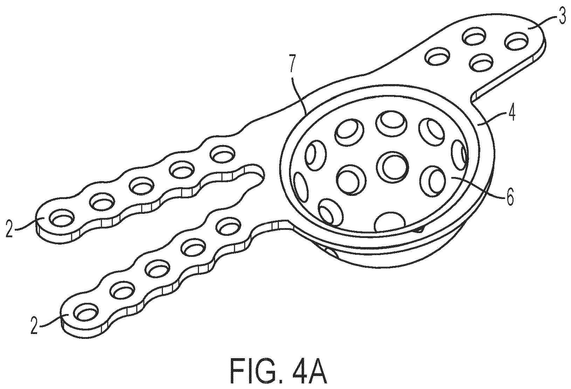

The socket 6 also includes an outer circumferential face 61, and an optional edge 60. In an inserted configuration (as shown in FIG. 4A), the circumferential face 61 of the socket-like support body 6 is maintained in contact with the fastening ring 4 or the fastening ring 104 by contact between the upper surface of the fastening ring 4 or an upper surface of fastening ring 104 and the optional edge 60.

The socket-like support body 6 can be made of a biocompatible material that is of sufficient strength so as to take up the bearing forces of the hip joint. TiA16V4 is one option of biocompatible material the socket-like support body 6 can be formed of.

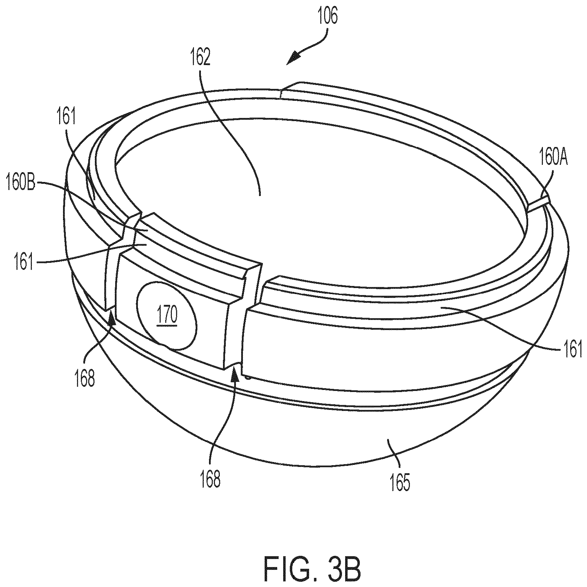

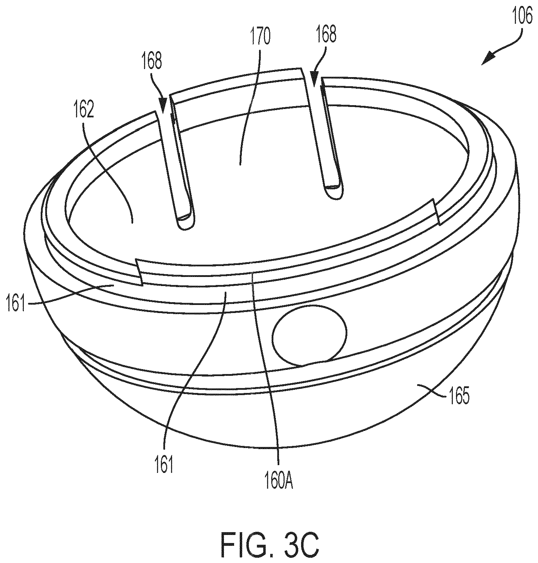

The socket 106, which can also be referred to as socket-like support body 106, or socket support body 106, is shown in FIGs. 3B and 3C and has a substantially hemispherical shape. The socket 106 includes a curved, dome shape and has a substantially flat, concave, curved inner surface 162. On a convex outer face 165, the socket-like support body 106 is configured to bear against the bone surfaces of the pelvic bone 9, for a limited time and not for implantation. The -like 106 can be used in either inner region 40 or inner region 140 during shaping of fastening brackets 102, 103, or during reshaping of fastening brackets 102, 103.

As the socket-like support body 106 is not configured for implantation, the socket-like support body 106 can be formed of any suitable biocompatible or non-biocompatible material, such as any metal(s), plastic(s), epoxy(s), rubber(s), cellulose based material(s), organic material(s) such as leather(s), fabric(s)(woven and/or non-woven), carbon based material(s), and combinations thereof.

The socket-like support body 106 is configured for relative ease of removal from either inner region 40 or inner region 140 as compared to the socket-like support body 6. This relative ease is based on the structure of the socket-like support body 106, such as distal edge 160A, a tab 170 and a proximal edge 160B on the tab 170. The tab 170 is separated from the other portions of the circumference of the socket-like support body 106 by two tab slots 168.

The distal edge 160A extends substantially orthogonally from circumferential face 161, with the distal edge 160A extending a portion of the circumference of the socket-like support body 106. The circumferential face 161 is configured to contact an inner circumferential face of the fastening ring 4 or an inner circumferential face of fastening ring 104. The distal edge 160A is configured to contact an upper surface of the fastening ring 4 or an upper surface of fastening ring 104.

The proximal edge 160B extends substantially orthogonally from circumferential face 161, with the proximal edge 160B extending at least a portion of the circumference width of the tab 170. The proximal edge 160B is configured to contact an upper surface of the fastening ring 4 or an upper surface of fastening ring 104.

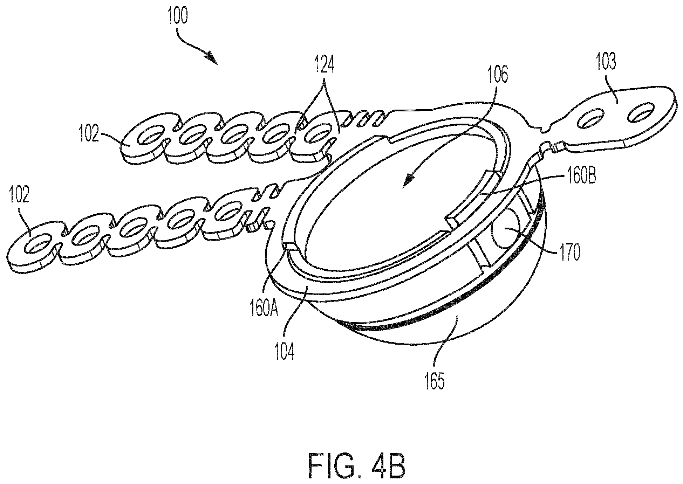

Thus, in an inserted configuration (as shown in FIG. 4B), the socket-like support body 106 is maintained in contact with the fastening ring 4 or the fastening ring 104 by contact between the upper surface of the fastening ring 4 or an upper surface of fastening ring 104 and both the distal edge 160A and the proximal edge 160B. To separate the socket-like support body 106 from the inserted configuration, a user can apply pressure to the tab 170, causing the proximal edge 160B to move towards a center of the socket-like support body 106 such that the proximal edge 160B does not contact an upper surface of the fastening ring 4 or an upper surface of fastening ring 104. The socket-like support body 106 can then be moved away from contact with the fastening ring 4 or the fastening ring 104.

Another view of the socket-like support body 106 is shown in FIG. 3C.

FIG. 4A shows socket-like support body 6 in an inserted configuration, inserted into the opening 40 (shown in FIG. 2A) of fastening ring 4. The socket-like support body 6 is inserted until the fastening ring 4, in the region of the upper end on the socket edge 59, engages with an relatively tight fit around the socket-like support body 6. In some examples, this relatively tight fit is an interference fit.

FIG. 4B shows the socket-like support body 106 in an inserted configuration, inserted into the opening 140 (shown in FIG. 2B) of fastening ring 104. The socket-like support body 106 is inserted such that the tab 170 moves towards a center of the socket-like support body 106, the proximal edge 160B then clears an upper edge of the fastening ring 104, the tab 170 moves away from a center of the socket-like support body 106 and the distal edge 160A and the proximal edge 160B contact an upper surface of the fastening ring 104.

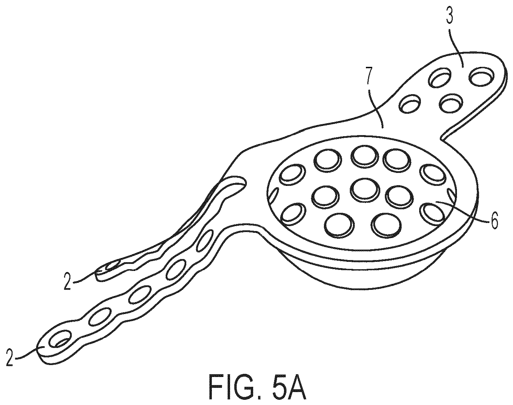

As the material of fastening brackets 2, and 3, and the material of fastening ring 4 is a reshapeable material, FIG. 5A shows those portions in a reshaped configuration, while socket-like support body 6 is in the inserted configuration. However, in other examples, fastening brackets 2, and 3, and the fastening ring 4 can be in a reshaped configuration, while socket-like support body 106 is in the inserted configuration. In either case, the contoured form the fastening brackets 2, and 3, and the fastening ring 4 take are based on a contour of the pelvic bone 9. In some examples a user can reshape the fastening brackets 2, and 3, and the fastening ring 4 based on a visual inspection of the pelvic bone 9, either by having the pelvic bone 9 exposed and/or upon review of a scan (such as an X-ray or Computerized Tomography (CT) scan). During implantation the fastening brackets 2, and 3, and the fastening ring 4 can optionally further be reshaped based on respective individual anatomy of the pelvic bone can 9.

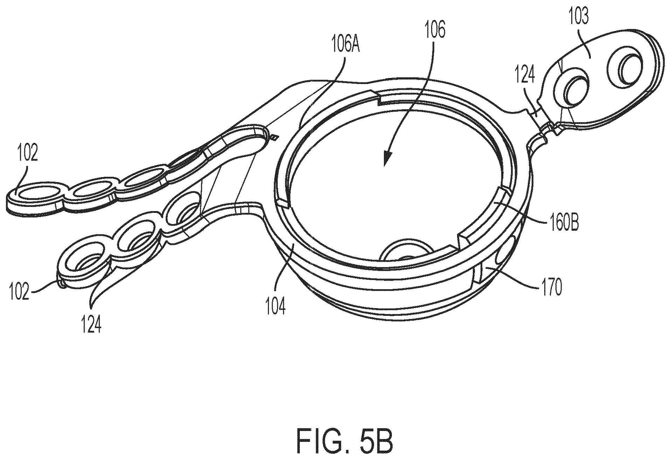

As the material of fastening brackets 102, and 103, and the material of fastening ring 104 is a reshapeable material, FIG. 5B shows those portions in a reshaped configuration, while socket-like support body 106 is in the inserted configuration. However, in other examples, fastening brackets 102, and 103, and the fastening ring 104 can be in a reshaped configuration, while socket-like support body 6 is in the inserted configuration. In either case, the contoured form the fastening brackets 102, and 103, and the fastening ring 104 take are based on a contour of the pelvic bone 9. In some examples a user can reshape the fastening brackets 102, and 103, and the fastening ring 104 based on a visual inspection of the pelvic bone 9, either by having the pelvic bone 9 exposed and/or upon review of a scan (such as an X-ray or Computerized Tomography (CT) scan). During a trial or temporary implantation the fastening brackets 102, and 103, and the fastening ring 104 can optionally further be reshaped based on respective individual anatomy of the pelvic bone can 9.

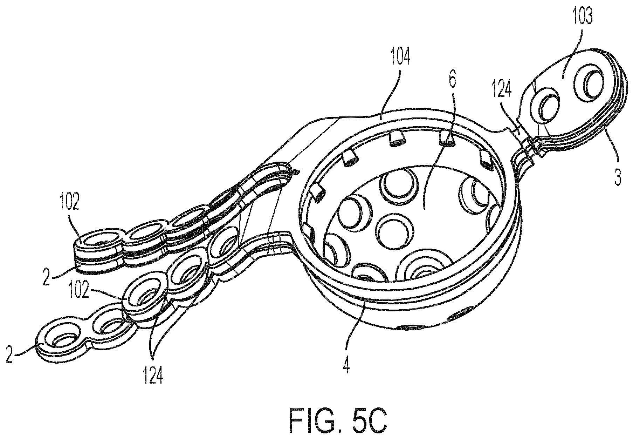

The fastening brackets 102, and 103, and the fastening ring 104 are comparatively easier to reshape than the fastening brackets 2, and 3, and the fastening ring 4 due to one or more of (1) the higher workability and ease of reshaping of the material of the fastening brackets 102, 103 and the fastening ring 104, and/or (2) inclusion of narrow portions 124 in each of the fastening brackets 102, 103 and the fastening ring 104. Thus, it is comparatively easier for a user to reshape the fastening brackets 102, and 103, and the fastening ring 104 into a configuration as shown in FIG. 5B as compared to reshaping fastening brackets 2, and 3, and the fastening ring 4 into a configuration as shown in FIG. 5A. Due to this relative ease, during use, a user can (1) first reshape the fastening brackets 102, and 103, and the fastening ring 104 into a configuration as shown in FIG. 5B (including either the socket-like support body 106 in the inserted configuration, or the socket-like support body 6 in the inserted configuration), (2) then remove either the socket-like support body 106 or the socket-like support body 6 from its inserted configuration, (3) then reshape a substantially flat fastening brackets 2, and 3, and the fastening ring 4 to substantially match the shape/orientation of the previously reshaped fastening brackets 102, and 103, and the fastening ring 104, and (4) then insert either the socket-like support body 106 or the socket-like support body 6 into the reshaped fastening brackets 2, and 3, and the fastening ring 4. For example, in step (4) if the socket-like support body 6 is to be inserted, the reshaped fastening brackets 2, and 3, and the fastening ring 4 will substantially match the shape/orientation of the previously reshaped fastening brackets 102, and 103, and the fastening ring 104, and the previously reshaped fastening brackets 102, and 103, and the fastening ring 104 can be overlaid onto the socket-like support body 6, the reshaped fastening brackets 2, and 3, and the fastening ring 4, as shown in FIG. 5C.

The advantage of using fastening brackets 102, and 103, and the fastening ring 104 as a guide for fastening brackets 2, and 3, and the fastening ring 4 (which will be implanted) is that manual reshaping of fastening brackets 2, and 3, and the fastening ring 4 can occur away from the patient who is to receive the implant's pelvic bone 9.

The described embodiments and examples of the present disclosure are intended to be illustrative rather than restrictive, and are not intended to represent every embodiment or example of the present disclosure. While the fundamental novel features of the disclosure as applied to various specific embodiments thereof have been shown, described and pointed out, it will also be understood that various omissions, substitutions and changes in the form and details of the devices illustrated and in their operation, may be made by those skilled in the art without departing from the scope of the invention, which is defined by the claims.

## Claims

1. A pelvic implantation device (100) comprising:
at least two outwardly directed, flat, fastening brackets (102); and
a socket support body (106) comprising:
a convex outer face (165) configured to contact a pelvic bone;
a concave inner face (162) configured as a receiving seat, the receiving seat configured to receive a joint head of a femoral component of a hip prosthesis,
wherein the fastening brackets are arranged at an edge of a fastening ring (104), the fastening ring forming an opening (140) configured to receive the socket support body, wherein each of the fastening brackets includes adjacent fastening openings (121) configured to receive fastening means and narrow portions (124) narrower than a width dimension (126) of the fastening bracket between two or more, or all of the adjacent fastening openings, **characterized in that** each of the fastening brackets includes one or more additional narrow portions (124) narrower than the width dimension of the fastening bracket, provided between the one fastening opening nearest the opening and the fastening ring.

2. The pelvic implantation device of claim 1, wherein the socket support body is made of a biocompatible material that is stiffer than a material of the fastening brackets.

3. The pelvic implantation device of any one of the preceding claims, wherein the socket support body is cohesively connected to the fastening ring by means of a cohesive bond such as a welded connection, preferably a welded-though weld seam.

4. The pelvic implantation device of any one of the preceding claims, wherein the fastening brackets comprise a reshapeable material that is biocompatible.

5. The pelvic implantation device of any one of the preceding claims, wherein the fastening brackets comprise a titanium.

6. The pelvic implantation device of any one of the preceding claims, wherein the fastening brackets are made in one piece with the fastening ring.

7. The pelvic implantation device of any one of the preceding claims, wherein the fastening ring is completely closed.

8. The pelvic implantation device of any one of claims 1 to 6, wherein the fastening ring is partially open.

9. The pelvic implantation device of any one of the preceding claims, wherein the fastening brackets comprise two cranially directed fastening brackets (102), the pelvic implantation device further comprising a caudally directed fastening bracket (103) provided on a side of the socket support body opposite the two cranially directed fastening brackets.

10. The pelvic implantation device of claim 9, wherein the caudally directed fastening bracket is shorter than the two cranially directed brackets.

11. The pelvic implantation device of claim 9, wherein the caudally directed fastening bracket is wider than the two cranially directed brackets.

12. The pelvic implantation device of any one of claims 1 to 11, wherein the socket support body comprises:
a distal edge (160A) that extends substantially orthogonally from a circumferential surface of the socket support body; and
a tab (170), wherein the tab forms a portion of the circumferential surface of the socket support body, the tab comprising a proximal edge (160B) that extends substantially orthogonally from a surface of the tab,
wherein the distal edge and the proximal edge are configured to contact an upper surface of the fastening ring.

13. The pelvic implantation device of any one of claims 1 to 11, wherein the socket support body comprises an edge (160A, 160B) that extends substantially orthogonally from a circumferential surface of the socket support body, wherein the edge is configured to provide a tight fit or an interference fit with the fastening ring.

## Patentansprüche

1. Beckenimplantationsvorrichtung (100), umfassend:
mindestens zwei nach außen gerichtete, flache Befestigungslaschen (102); und
einen Pfannentragkörper (106), umfassend:
eine konvexe Außenfläche (165), die so konfiguriert ist, dass sie einen Beckenknochen kontaktiert;
eine konkave Innenfläche (162), die als Aufnahmesitz konfiguriert ist, wobei der Aufnahmesitz so konfiguriert ist, dass er einen Gelenkkopf einer Femurkomponente einer Hüftprothese aufnimmt,
wobei die Befestigungslaschen an einem Rand eines Befestigungsrings (104) angeordnet sind, wobei der Befestigungsring eine Öffnung (140) ausbildet, die so konfiguriert ist, dass sie den Pfannentragkörper aufnimmt, wobei jede der Befestigungslaschen benachbarte Befestigungsöffnungen (121), die so konfiguriert sind, dass sie Befestigungsmittel aufnehmen, und schmale Abschnitte (124) einschließt, die schmaler als ein Breitenmaß (126) der Befestigungslasche zwischen zwei oder mehr oder allen der benachbarten Befestigungsöffnungen sind, **dadurch gekennzeichnet, dass** jede der Befestigungslaschen einen oder mehrere zusätzliche schmale Abschnitte (124) einschließt, die schmaler als das Breitenmaß der Befestigungslasche sind und zwischen der einen, der Öffnung am nächsten liegenden Befestigungsöffnung und dem Befestigungsring bereitgestellt sind.

2. Beckenimplantationsvorrichtung nach Anspruch 1, wobei der Pfannentragkörper aus einem biokompatiblen Material hergestellt ist, das steifer ist als ein Material der Befestigungslaschen.

3. Beckenimplantationsvorrichtung nach einem der vorhergehenden Ansprüche, wobei der Pfannentragkörper mit dem Befestigungsring mittels einer kohäsiven Verbindung, wie beispielsweise einer Schweißverbindung, vorzugsweise einer durchgeschweißten Schweißnaht, kohäsiv verbunden ist.

4. Beckenimplantationsvorrichtung nach einem der vorhergehenden Ansprüche, wobei die Befestigungslaschen ein umformbares Material umfassen, das biokompatibel ist.

5. Beckenimplantationsvorrichtung nach einem der vorhergehenden Ansprüche, wobei die Befestigungslaschen ein Titan umfassen.

6. Beckenimplantationsvorrichtung nach einem der vorhergehenden Ansprüche, wobei die Befestigungslaschen einstückig mit dem Befestigungsring ausgebildet sind.

7. Beckenimplantationsvorrichtung nach einem der vorhergehenden Ansprüche, wobei der Befestigungsring vollständig geschlossen ist.

8. Beckenimplantationsvorrichtung nach einem der Ansprüche 1 bis 6, wobei der Befestigungsring teilweise offen ist.

9. Beckenimplantationsvorrichtung nach einem der vorhergehenden Ansprüche, wobei die Befestigungslaschen zwei kranial gerichtete Befestigungslaschen (102) umfassen, wobei die Beckenimplantationsvorrichtung des Weiteren umfasst
eine kaudal gerichtete Befestigungslasche (103), die auf einer Seite des Pfannentragkörpers bereitgestellt ist, die den beiden kranial gerichteten Befestigungslaschen gegenüberliegt.

10. Beckenimplantationsvorrichtung nach Anspruch 9, wobei die kaudal gerichtete Befestigungslasche kürzer ist als die beiden kranial gerichteten Laschen.

11. Beckenimplantationsvorrichtung nach Anspruch 9, wobei die kaudal gerichtete Befestigungslasche breiter ist als die beiden kranial gerichteten Laschen.

12. Beckenimplantationsvorrichtung nach einem der Ansprüche 1 bis 11, wobei der Pfannentragkörper umfasst:
einen distalen Rand (160A), der sich im Wesentlichen orthogonal von einer Umfangsfläche des Pfannentragkörpers erstreckt; und
eine Lasche (170), wobei die Lasche einen Abschnitt der Umfangsfläche des Pfannentragkörpers ausbildet, wobei die Lasche einen proximalen Rand (160B) umfasst, der sich im Wesentlichen orthogonal von einer Fläche der Lasche erstreckt,
wobei der distale Rand und der proximale Rand so konfiguriert sind, dass sie eine obere Fläche des Befestigungsrings kontaktieren.

13. Beckenimplantationsvorrichtung nach einem der Ansprüche 1 bis 11, wobei der Pfannentragkörper einen Rand (160A, 160B) umfasst, der sich im Wesentlichen orthogonal von einer Umfangsfläche des Pfannentragkörpers erstreckt, wobei der Rand so konfiguriert ist, dass er einen Festsitz oder einen Presssitz mit dem Befestigungsring bereitstellt.

## Revendications

1. Dispositif d'implantation pelvienne (100) comprenant :
au moins deux supports de fixation plats, dirigés vers l'extérieur (102) ; et
un corps de support de douille (106) comprenant :
une face extérieure convexe (165) configurée pour entrer en contact avec un os pelvien ;
une face intérieure concave (162) configurée comme un siège de réception, le siège de réception étant configuré pour recevoir une tête d'articulation d'un composant fémoral d'une prothèse de hanche,
dans lequel les supports de fixation sont disposés sur un bord d'une bague de fixation (104), la bague de fixation formant une ouverture (140) configurée pour recevoir le corps de support de douille, dans lequel chacun des supports de fixation comporte des ouvertures de fixation adjacentes (121) configurées pour recevoir des moyens de fixation et des parties étroites (124) plus étroites qu'une dimension de largeur (126) du support de fixation entre deux ou plusieurs, ou toutes les ouvertures de fixation adjacentes, **caractérisé en ce que** chacun des supports de fixation comporte une ou plusieurs parties étroites supplémentaires (124) plus étroites que la dimension de largeur du support de fixation, prévues entre l'ouverture de fixation la plus proche de l'ouverture et la bague de fixation.

2. Dispositif d'implantation pelvienne selon la revendication 1, dans lequel le corps de support de douille est constitué d'un matériau biocompatible qui est plus rigide qu'un matériau des supports de fixation.

3. Dispositif d'implantation pelvienne selon l'une quelconque des revendications précédentes, dans lequel le corps de support de douille est relié de manière cohésive à la bague de fixation au moyen d'une liaison cohésive telle qu'une liaison soudée, de préférence un cordon de soudure traversant.

4. Dispositif d'implantation pelvienne selon l'une quelconque des revendications précédentes, dans lequel les supports de fixation comprennent un matériau remodelable qui est biocompatible.

5. Dispositif d'implantation pelvienne selon l'une quelconque des revendications précédentes, dans lequel les supports de fixation comprennent du titane.

6. Dispositif d'implantation pelvienne selon l'une quelconque des revendications précédentes, dans lequel les supports de fixation sont fabriqués d'un seul tenant avec la bague de fixation.

7. Dispositif d'implantation pelvienne selon l'une quelconque des revendications précédentes, dans lequel la bague de fixation est complètement fermée.

8. Dispositif d'implantation pelvienne selon l'une quelconque des revendications 1 à 6, dans lequel la bague de fixation est partiellement ouverte.

9. Dispositif d'implantation pelvienne selon l'une quelconque des revendications précédentes, dans lequel les supports de fixation comprennent deux supports de fixation dirigés crânialement (102), le dispositif d'implantation pelvienne comprenant également
un support de fixation dirigé caudalement (103) prévu sur un côté du corps de support de douille opposé aux deux supports de fixation dirigés crânialement.

10. Dispositif d'implantation pelvienne selon la revendication 9, dans lequel le support de fixation dirigé caudalement est plus court que les deux supports dirigés crânialement.

11. Dispositif d'implantation pelvienne selon la revendication 9, dans lequel le support de fixation dirigé caudalement est plus large que les deux supports dirigés crânialement.

12. Dispositif d'implantation pelvienne selon l'une quelconque des revendications 1 à 11, dans lequel le corps de support de douille comprend :
un bord distal (160A) qui se prolonge sensiblement orthogonalement à partir d'une surface circonférentielle du corps de support de douille ; et
une languette (170), dans lequel la languette forme une partie de la surface circonférentielle du corps de support de douille, la languette comprenant un bord proximal (160B) qui se prolonge sensiblement orthogonalement à partir d'une surface de la languette,
dans lequel le bord distal et le bord proximal sont configurés pour entrer en contact avec une surface supérieure de la bague de fixation.

13. Dispositif d'implantation pelvienne selon l'une quelconque des revendications 1 à 11, dans lequel le corps de support de douille comprend un bord (160A, 160B) qui se prolonge sensiblement orthogonalement à partir d'une surface circonférentielle du corps de support de douille, dans lequel le bord est configuré pour fournir un ajustement serré ou un ajustement par interférence avec la bague de fixation.
